# EUROPEAN PATENT SPECIFICATION

(11) **EP 1 829 221 B1**
(45) Date of publication and mention of the grant of the patent: **23.07.2008**
(21) Application number: 04822474.5
(22) Date of filing: 24.11.2004
(51) Int. Cl.: H03M 7/00

(54) **ATTACHMENT OF TUBING IN A CARDIAC LEAD**
ANBRINGUNG VON SCHLÄUCHEN IN EINER HERZLEITUNG
FIXATION D'UNE TUBULURE DANS UNE SONDE CARDIAQUE

(43) Date of publication of application: 05.09.2007
(73) Proprietor: St. Jude Medical AB, 175 84 Järfälla (SE)
(72) Inventor: HALLANDER, Sara, SE-125 40 Älvsjö (SE); NILSSON, Susanne, S-141 33 Huddinge (SE); DOWLING, Kenneth, S-197 91 Bro (SE); MICSKI, Eva, S-14143 Huddinge (SE); ERIKSSON, Tom, S-81132 Sanviken (SE)
(86) International application number: PCT/SE2004/001738
(87) International publication number: WO 2006/057582

(56) References cited:
- EP-A2- 1 254 916
- US-A- 5 014 720
- US-A- 5 354 327
- US-A1- 2002 143 377
- US-A1- 2003 050 528
- US-A1- 2004 054 210

## Description

### Technical field of the invention

The present invention relates to a cardiac lead comprising a thermoplastic tubing and an electrode or a pin connector, and to a method for fixing a thermoplastic cardiac lead tubing to a cardiac lead electrode or pin connector.

### Background art

A cardiac lead may be a bipolar (or unipolar or multipolar) electrode lead used for providing stimulation of cardiac tissue, and/or for sensing heart signals, by means of a pulse generator or some other type of heart stimulation apparatus. The cardiac lead carries the stimulus from the pulse generator to the cardiac tissue, or relays intrinsic cardiac signals back to a sense amplifier of such pulse generator.

In a known cardiac lead, an electrically conducting coil interconnects a cardiac electrode (mounted at a distal end of the lead) and a connector (for connection to a cardiac stimulation device). Said coil is surrounded by a tubing (for protection and electrical insulation of the coil) that is attached to the electrode and the connector, respectively, at the ends of the cardiac lead. The tubing material is commonly silicone, which is advantageously fixed to the electrode or connector by a silicone adhesive. Such adhesives are commonly used in medical applications.

A desire for improved tubing properties, such as increased resistance to abrasion and altered flexibility, has lead to the use of other tubing material, such as organic polymeric materials. However, due to their intrinsic properties such polymeric materials cannot be effectively fixed to the electrode or connector by silicone adhesives. Other adhesives which may render effective fixation are known. These are, however, not approved for medical use. As an alternative, the solvent method has been applied for joining the tubing to the electrode. Disadvantageously, joints obtainable by said method are difficult to make accurately and also difficult to inspect.

US-A-5 354 327 discloses a cardiac lead comprising a thermoplastic tubing and an electrode or a pin connector, wherein said electrode is provided with outer fixation means and an end portion of the tubing is coaxially provided around the fixation means such that the tubing is fixed to the electrode.

### Summary of the invention

An object of the present invention is to provide an alternative, improved cardiac lead, wherein a thermoplastic tubing is attached to an electrode or a pin connector.

Another object of the present invention is to provide an alternative and improved method for attaching a thermoplastic tubing to an electrode or a pin connector.

The above-mentioned and other objects, which will become apparent in the following, are achieved by a cardiac lead as defined in claim 1 and by a method according to claim 13.

In the dependent claims there are stated additional features developing the invention further, and being characteristic of various preferred embodiments thereof.

The invention is based on the insight that the intrinsic properties of thermoplastic tubing materials can be utilised for their attachment to electrical contact means, such as an electrode or a pin connector, without the use of glues or adhesives. Hence, improved strength and simple production processes are achieved. Furthermore, no separate adhesive is necessary, thereby reducing costs and avoiding regulatory procedures concerning materials for use in medical devices.

Thus, in a first aspect of the present invention is provided a cardiac lead comprising a thermoplastic tubing and an electrode or a pin connector, wherein said electrode or a pin connector is provided with outer thermoplastic fixation means, an end portion of the tubing is coaxially provided around a portion of the fixation means, and a section of the end portion of the tubing is fused to the fixation means, such that the tubing is fixed to the electrode or pin connector.

Said fusing of the tubing and the fixation means provides a "seamless" attachment between the tubing and the electrode or pin connector, conferring mechanical strength, as well as an insulating closure.

The fixation means is preferably provided in a recess in the electrode or pin connector. Such fixation means can easily be casted during production of the contact means and provides for a durable attachment. Although not necessary, for the ease of production and quality of the attachment said thermoplastic fixation means may extend essentially around the perimeter of the electrical contact means.

In a second aspect of the present invention is provided a method for fixing a thermoplastic cardiac lead tubing to a cardiac lead electrode or pin connector provided with outer thermoplastic fixation means, said method comprising the steps of
a) positioning an end portion of the tubing coaxially around a portion of the fixation means;
b) heating at least a section of the end portion of the tubing and at least a portion of the fixation means, so as to achieve fusing of said heated section of the end portion of the tubing to said heated portion of the fixation means; and
c) allowing the resulting fused portion to solidify.

Said method results in a cardiac lead according to the first aspect of the present invention. The heating of the thermoplastic tubing and fixation means material to a temperature close to the melting points thereof causes fusing of the materials. The fused materials form a "seamless" connection when allowed to solidify.

Said heating can be performed by any conventional means and preferably by conductive, irradiative or ultrasonic heating. More preferably the heating and fusing are performed by heat welding, providing a fast and reliable fixation of the tubing to the electrical contact means. Most preferably, the heat is applied locally to said section of the end portion of the tubing and to the fixation means, so as to avoid possibly negative influence on other parts of the cardiac lead. For example, the distal end of a cardiac lead may comprise a steroid plug, which is sensible to heat. Irradiative heating may be performed by laser. Ultrasonic heating may be performed as ultrasonic welding.

Supplementary heat treatment, such as annealing (e.g. to avoid environmental stress cracking), may additionally be performed.

The design of said electrode or pin connector is preferably as defined in relation to the first aspect of the invention.

In both aspects of the invention, the material of the thermoplastic tubing, as well as the material of the thermoplastic fixation means, preferably comprises a thermoplastic polymer. Such a material allows for heating to cause melting and for fusing, respectively. Property-wise such materials should preferably be rigid or flexible thermoplastic materials suitable for extrusion or injection molding and be biocompatible. Common materials fulfilling these requirements are polyurethanes, polyolefins (such as UHMWPE, HDPE, LDPE, etc.), polycarbonates (plexiglass), polyesters (such as PET, Dacron), polyamides (such as Nylon), polyimides, etc., the common characteristics being that the materials can be melted, reformed while in the molten state, and then become solid again in the new form upon cooling.

Generally, to facilitate fusing and to allow a durable joint, the melting point, melt index and/or hardness of the thermoplastic polymer of the two materials should preferably be close to each other. Furthermore, both materials should preferably be free from impurities. In particular, moisture and soot (resulting from e.g. insufficient cleaning of equipment used) should be avoided.

With regard to the above-mentioned general guidelines for the choice of materials and for fusing of thermoplastic polymers, preferred materials are characterized by having soft and hard segments in the polymer backbone, joined by reaction of isocyanates. Hence, any type of modified thermoplastic polyurethane material, such as a polyether polyurethane, a polycarbonate polyurethane, a poly(siloxane-carbonate) polyurethane, or a poly(ether-siloxane) polyurethane, may preferably be used for either of the thermoplastic tubing or the thermoplastic fixation means.

Depending on the general properties required for the tubing, such as resistance to abrasion, flexibility, durability and surface properties, a preferred thermoplastic polymer is a polyether polyurethane comprising soft segments and hard segments or a poly(ether-siloxane) polyurethane comprising soft segments and hard segments. In a polyether polyurethane, the soft segments may comprise polytetramethyleneoxide (PTMO) (such as in Pellethane^{™}, Dow Chemical Company) or polyhexamethyleneoxide (PHMO). A preferred polyether polyurethane is a Pellethane^{™}, more preferred is Pellethane^{™} 2363-55D. In a poly(ether-siloxane) polyurethane, the soft segments may comprise polyhexamethyleneoxide (PHMO) and polydimethylsiloxane (PDMS) (such as in Elast-Eon^{™}). A preferred poly(ether-siloxane) polyurethane is an Elast-Eon^{™}, more preferred is Elast-Eon^{™} 2A. The hard segments of the polyether polyurethane or the poly(ether-siloxane) polyurethane, respectively, may comprise a diisocyanate, such as methylenediphenyldiisocyanate (MDI), and a diol, such as butanediol (BDO), as a chain extender.

Depending on the properties required for the fixations means, such as compatibility with the tubing, castability, melting point etc., a preferred thermoplastic polymer is a polyether polyurethane comprising soft segments and hard segments. The soft segments may comprise polytetramethylenedioxide (PTMO) (such as in Tecothane®, Thermedics Polymer Products) or polyhexamethylenedioxide (PHMO). The hard segments may comprise a diisocyanate, such as methylenediphenyldiisocyanate (MDI) and a diol, such as butanediol (BDO), as a chain extender. A preferred polyether polyurethane is a Tecothane®, more preferred is Tecothane® 1075D.

### Brief description of the drawing

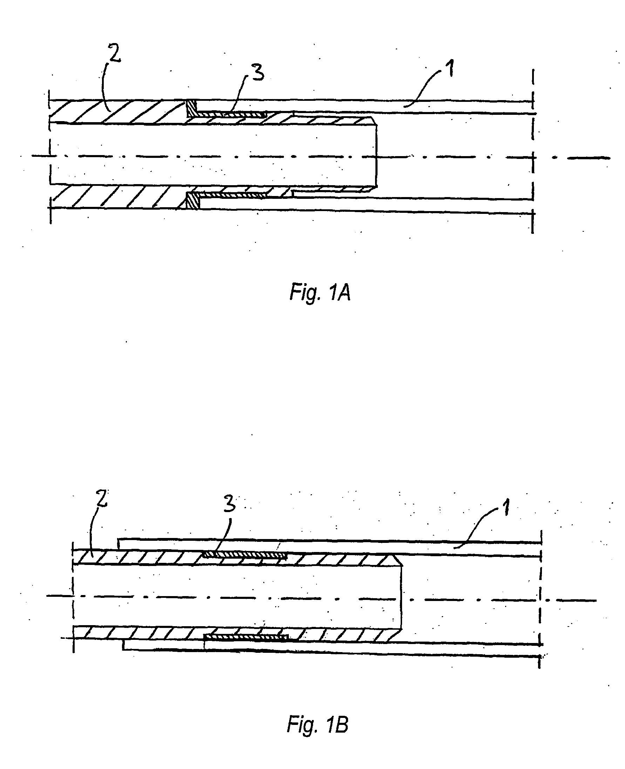

Fig. 1 comprises cross-sectional views showing schematically the fixing of a thermoplastic tubing material to an electrode or a pin connector provided with outer thermoplastic fixation means by fusion. Figs. 1A and 1B represent different embodiments of said fixing.

### Detailed description of preferred embodiments

In a cardiac lead according to Fig. 1A, a tubing 1 was fixed to a ring electrode 2. The ring electrode had been provided with a fixation means 3. The tubing was fixed to the ring electrode by heat welding of the tubing to the fixation means, i.e. two semicircular jaws clamped and heated the arrangement of tubing and fixation means to obtain a joint.

Another embodiment of a tubing 1 fixed to an electrode 2 provided with a fixation means 3 is shown in Fig. 1B.

### Example

Tensile testing of cardiac leads according to the embodiment shown in Fig. 1A was performed according to EN45502-2-1 (CEN/CENELEC). The electrode was made of Pt/Ir 90/10 and was provided with a fixation means made of Tecothane® 1075D, which had been injection moulded onto the electrode. The fixation means of 0.1 mm thickness and 1.4 mm length was provided in a 0.1 mm recess of 1.4 mm length extending around the perimeter of the electrode (1.55 mm diameter).

Three tubing materials were tested:
1) Elast-Eon^{™} 2A, inner diameter 1.70±0.025 mm, outer diameter 1.88±0.025 mm;
2) Elast-Eon^{™} 2A, inner diameter 1.70±0.025 mm, outer diameter 1.95±0.025 mm; and
3) Pellethane^{™} 2363-55D, inner diameter 1.69±0.05 mm, outer diameter 2.14±0.05 mm.

The heat welding was performed according to two different methods:
A) Two stage process comprising
   - welding at 135 °C for 5 s (jaws of unhardened tool steel) (fusion of tubing to fixation means) and
   - compression at 110 °C for 8 s (jaws of unhardened tool steel); and
B) Welding at 240 °C for 40 s (jaws of unhardened tool steel with a teflon surface).

Each group tested comprised 8-13 samples. Pull force results (N) at break are presented as mean values and standard deviation in Table 1.

**Table 1. Tensile testing, pull force**

| Tubing material | Heat welding | Mean value (N) | Standard deviation |
|---|---|---|---|
| 1 | A | 10.8 | 1.9 |
| 2 | A | 9.0 | 0.8 |
| 2 | B | 9.5 | 2.4 |
| 3 | A | 14.4 | 2.3 |

Safe cardiac lead performance requires (EN45502-2-1 (CEN/CENELEC)) the cardiac lead to withstand 5 N pull force during 1 minute. All samples complied with this requirement. Furthermore, the pull force mean value at fracture was ≥ 9 N. All samples present a small standard deviation, thus being suitable for accurate production.

## Claims

1. A cardiac lead comprising a thermoplastic tubing (1) and an electrode (2) or a pin connector, wherein said electrode or pin connector is provided with outer thermoplastic fixation means (3), an end portion of the tubing is coaxially provided around at least a portion of the fixation means, and a section of the end portion of the tubing is fused to the fixation means, such that the tubing is fixed to the electrode or pin connector.

2. A cardiac lead according to claim 1, wherein the thermoplastic fixation means is provided in a recess in the electrode or pin connector.

3. A cardiac lead according to claim 1 or 2, wherein the thermoplastic fixation means extends substantially around the perimeter of the electrode or pin connector.

4. A cardiac lead according to any one of the preceding claims, wherein the material of the thermoplastic fixation means and/or the thermoplastic tubing comprises a polyurethane, a polyolefin, a polycarbonate, a polyester, a polyamide or a polyimide.

5. A cardiac lead according to claim 4, wherein the material of the thermoplastic fixation means and/or the thermoplastic tubing comprises a polyether polyurethane, a polycarbonate polyurethane, a poly(siloxane-carbonate) polyurethane or a poly(ether-siloxane) polyurethane.

6. A cardiac lead according to any one of the preceding claims, wherein the material of the thermoplastic tubing comprises a polyether polyurethane comprising soft segments and hard segments.

7. A cardiac lead according to claim 6, wherein the soft segments comprise polytetramethyleneoxide or polyhexamethyleneoxide.

8. A cardiac lead according to any one of claims 1 to 5, wherein the material of the thermoplastic tubing comprises a poly(ether-siloxane) polyurethane comprising soft segments and hard segments.

9. A cardiac lead according to claim 8, wherein the soft segments comprise polyhexamethyleneoxide and polydimethylsiloxane.

10. A cardiac lead according to any one of the preceding claims, wherein the material of the thermoplastic fixations means comprises a polyether polyurethane comprising soft segments and hard segments.

11. A cardiac lead according to claim 10, wherein the soft segments comprise polytetramethyleneoxide or polyhexamethyleneoxide

12. A cardiac lead according to any one of claims 6 to 11, wherein the hard segments comprise a diisocyanate and a diol.

13. A method for fixing a thermoplastic cardiac lead tubing to a cardiac lead electrode or pin connector provided with outer thermoplastic fixation means, said method comprising the steps of
a) positioning an end portion of the tubing coaxially around at least a portion of the fixation means;
b) heating at least a section of the end portion of the tubing and at least a portion of the fixation means, so as to achieve fusing of said heated section of the end portion of the tubing to said heated portion of the fixation means; and
c) allowing the resulting fused portion to solidify.

14. A method according to claim 13, wherein said heating is performed by conductive, irradiative or ultrasonic heating.

15. A method according to claim 13, wherein said heating and fusing are performed by heat welding.

16. A method according to any one of claims 13 to 15, wherein the thermoplastic fixation means is provided in a recess in the electrode or pin connector.

17. A method according to any one of claims 13 to 16, wherein the thermoplastic fixation means extends substantially around the perimeter of the electrode or pin connector.

18. A method according to any one of claims 13 to 17, wherein the material of the thermoplastic fixation means and/or the thermoplastic tubing comprises a polyurethane, a polyolefin, a polycarbonate, a polyester, a polyamide or a polyimide.

19. A method according to claim 18, wherein the material of the thermoplastic fixation means and/or the thermoplastic tubing comprises a polyether polyurethane, a polycarbonate polyurethane, a poly(siloxane-carbonate) polyurethane or a poly(ether-siloxane) polyurethane.

20. A method according to any one of claims 13 to 19, wherein the material of the thermoplastic tubing comprises a polyether polyurethane comprising soft segments and hard segments.

21. A method according to claim 20, wherein the soft segments comprise polytetramethyleneoxide or polyhexamethyleneoxide.

22. A method according to any one of claims 13 to 19, wherein the material of the thermoplastic tubing comprises a poly(ether-siloxane) polyurethane comprising soft segments and hard segments.

23. A method according to claim 22, wherein the soft segments comprise polyhexamethyleneoxide and polydimetylsiloxane.

24. A method according to any one of claims 13 to 23, wherein the material of the thermoplastic fixations means comprises a polyether polyurethane comprising soft segments and hard segments.

25. A method according to claim 24, wherein the soft segments comprise polytetramethyleneoxide or polyhexamethyleneoxide.

26. A method according to any one of claims 20 to 25, wherein the hard segments comprise a diisocyanate and a diol.

## Patentansprüche

1. Herzleitung, enthaltend ein thermoplastisches Schlauchmaterial (1) und eine Elektrode (2) oder einen Steckverbinder, wobei die genannte Elektrode bzw. der Steckverbinder mit einer äußeren thermoplastischen Befestigungsvorrichtung (3) versehen, ein Endbereich des Schlauchmaterials koaxial um wenigstens einen Teil der Befestigungsvorrichtung angebracht und ein Abschnitt des Endteils des Schlauchmaterials mit der Befestigungsvorrichtung verschmolzen ist, so dass das Schlauchmaterial an der Elektrode bzw. dem Steckverbinder befestigt ist.

2. Herzleitung nach Anspruch 1, bei der die thermoplastische Befestigungsvorrichtung in einem Ausschnitt der Elektrode oder des Steckverbinders vorgesehen ist.

3. Herzleitung nach Anspruch 1 oder 2, bei der sich die thermoplastische Befestigungsvorrichtung im Wesentlichen um den Umfang der Elektrode oder des Steckverbinders erstreckt.

4. Herzleitung nach einem der vorhergehenden Ansprüche, bei der der Stoff der thermoplastischen Befestigungsvorrichtung und/oder des thermoplastischen Schlauchmaterials ein Polyurethan, ein Polyolefin, ein Polycarbonat, einen Polyester, ein Polyamid oder ein Polyimid enthält.

5. Herzleitung nach Anspruch 4, bei der der Stoff der thermoplastischen Befestigungsvorrichtung und/oder des thermoplastischen Schlauchmaterials ein Polyether Polyurethan, ein Polycarbonat Polyurethan, ein Poly(Siloxan-Carbonat) Polyurethan oder ein Poly(Ether-Siloxan) Polyurethan enthält.

6. Herzleitung nach einem der vorhergehenden Ansprüche, bei der der Stoff des thermoplastischen Schlauchmaterials ein Polyether Polyurethan mit weichen und harten Abschnitten enthält.

7. Herzleitung nach Anspruch 6, bei der die weichen Abschnitte ein Polytetramethylenoxid oder ein Polyhexamethylenoxid enthalten.

8. Herzleitung nach einem der Ansprüche 1 bis 5, bei der der Stoff des thermoplastischen Schlauchmaterials ein Poly(Ether-Siloxan) Polyurethan mit weichen und harten Abschnitten enthält.

9. Herzleitung nach Anspruch 8, bei der die weichen Abschnitte Polyhexamethylenoxid und Polydimethylsiloxan enthalten.

10. Herzleitung nach einem der vorhergehenden Ansprüche, bei der das Material der thermoplastischen Befestigungsvorrichtung ein Polyetherpolyurethan mit weichen und harten Abschnitten enthält.

11. Herzleitung nach Anspruch 10, bei der die weichen Abschnitte Polytetramethylenoxid oder Polyhexamethylenoxid enthalten.

12. Herzleitung nach einem der Ansprüche 6 bis 11, bei der die harten Abschnitte ein Diisozyanat und ein Diol enthalten.

13. Verfahren zum Befestigen eines thermoplastischen Herzleitungsschlauchmaterials an einer Herzleitungselektrode oder einem Steckverbinder, die/der mit einer äußeren thermoplastischen Befestigungsvorrichtung versehen ist, wobei das genannte Verfahren die folgenden Schritte umfasst
a) Positionieren eines Endbereiches des Schlauchmaterials koaxial um wenigstens einen Teil der Befestigungsvorrichtung;
b) Erhitzen wenigstens eines Abschnittes des Endbereiches des Schlauchmaterials und wenigstens eines Teils der Befestigungsvorrichtung so, dass ein Verschmelzen des genannten erhitzten Abschnittes des Endbereiches des Schlauchmaterials mit dem genannten erhitzten Teil der Befestigungsvorrichtung erreicht wird; und
c) Zulassen, dass sich der erhaltene geschmolzene Abschnitt verfestigt.

14. Verfahren nach Anspruch 13, bei dem das genannte Erhitzen durch konduktives Erhitzen, Strahlungshitze oder Erhitzen mit Ultraschall durchgeführt wird.

15. Verfahren nach Anspruch 13, bei dem das genannte Erhitzen und Verschmelzen durch Heißschweißen durchgeführt wird.

16. Verfahren nach einem der Ansprüche 13 bis 15, bei dem die thermoplastische Befestigungsvorrichtung in einem Ausschnitt in der Elektrode oder in dem Steckverbinder vorgesehen ist.

17. Verfahren nach einem der Ansprüche 13 bis 16, bei dem sich die thermoplastische Befestigungsvorrichtung im Wesentlichen um den Umfang der Elektrode oder des Steckverbinders erstreckt.

18. Verfahren nach einem der Ansprüche 13 bis 17, bei dem der Stoff der thermoplastischen Befestigungsvorrichtung und/oder des thermoplastischen Schlauchmaterials ein Polyurethan, ein Polyolefin, ein Polycarbonat, einen Polyester, ein Polyamid oder ein Polyimid enthält.

19. Verfahren nach Anspruch 18, bei dem der Stoff der thermoplastischen Befestigungsvorrichtung und/oder des thermoplastischen Schlauchmaterials ein Polyether Polyurethan, ein Polycarbonat Polyurethan, ein Poly(Siloxan-Carbonat) Polyurethan oder ein Poly(Ether-Siloxan) Polyurethan enthält.

20. Verfahren nach einem der Ansprüche 13 bis 19, bei dem der Stoff des thermoplastischen Schlauchmaterials ein Polyether Polyurethan mit weichen und harten Abschnitten enthält.

21. Verfahren nach Anspruch 20, bei dem die weichen Abschnitte Polytetramethylenoxid oder Polyhexamethylenoxid enthalten.

22. Verfahren nach einem der Ansprüche 13 bis 19, bei dem der Stoff des thermoplastischen Schlauchmaterials ein Poly(Ether-Siloxan) Polyurethan mit weichen und harten Abschnitten enthält.

23. Verfahren nach Anspruch 22, bei dem die weichen Abschnitte Polyhexamethylenoxid und Polydimethylsiloxan enthalten.

24. Verfahren nach einem der Ansprüche 13 bis 23, bei dem der Stoff der thermoplastischen Befestigungsvorrichtung ein Polyether Polyurethan mit weichen und harten Abschnitten enthält.

25. Verfahren nach Anspruch 24, bei dem die weichen Abschnitte Polytetramethylenoxid oder Polyhexamethylenoxid enthalten.

26. Verfahren nach einem der Ansprüche 20 bis 25, bei dem die harten Abschnitte ein Diisozyanat und ein Diol enthalten.

## Revendications

1. Dérivation cardiaque comprenant une tubulure ( 1 ) thermoplastique et une électrode ( 2 ) ou un connecteur à broche, l'électrode ou le connecteur à broche étant muni de moyens ( 3 ) de fixation extérieurs thermoplastiques, un tronçon d'extrémité de la tubulure étant prévu coaxialement autour d'au moins un tronçon des moyens de fixation et une partie du tronçon d'extrémité de la tubulure étant assemblé par fusion aux moyens de fixation, de manière à ce que la tubulure soit fixée à l'électrode ou au connecteur à broche.

2. Dérivation cardiaque suivant la revendication 1, dans laquelle les moyens de fixation thermoplastiques sont prévus dans une cavité de l'électrode ou du connecteur à broche.

3. Dérivation cardiaque suivant la revendication 1 ou 2, dans laquelle les moyens de fixation thermoplastiques s'étendent sensiblement autour du périmètre de l'électrode ou du connecteur à broche.

4. Dérivation cardiaque suivant l'une quelconque des revendications précédentes, dans laquelle la matière des moyens de fixation thermoplastiques et/ou de la tubulure thermoplastique comprend un polyuréthane, une polyoléfine, un polycarbonate, un polyester, un polyamide ou un polyimide.

5. Dérivation cardiaque suivant la revendication 4, dans laquelle la matière des moyens de fixation thermoplastiques et/ou de la tubulure thermoplastique comprend un polyéther polyuréthane, un polycarbonate polyuréthane, un poly( siloxane-carbonate ) polyuréthane ou un poly( éther-siloxane ) polyuréthane.

6. Dérivation cardiaque suivant l'une quelconque des revendications précédentes, dans laquelle la matière de la tubulure thermoplastique comprend un polyéther polyuréthane ayant des segments souples et des segments durs.

7. Dérivation cardiaque suivant la revendication 6, dans laquelle les segments souples comprennent du poly( oxyde de tétraméthylène ) ou du poly( oxyde d'hexaméthylène ).

8. Dérivation cardiaque suivant l'une quelconque des revendications précédentes, dans laquelle la matière de la tubulure thermoplastique comprend du poly(éther siloxane) polyuréthane comprenant des segments souples et des segments durs.

9. Dérivation cardiaque suivant la revendication 8, dans laquelle les segment souples comprennent du poly(oxyde d'hexaméthylène) et du polydiméthylsiloxane.

10. Dérivation cardiaque suivant l'une quelconque des revendications précédentes, dans laquelle la matière des moyens de fixation thermoplastiques comprend un polyéther polyuréthane comprenant des segments souples et des segments durs.

11. Dérivation cardiaque suivant la revendication 10, dans laquelle les segments souples comprennent du poly( oxyde de tétraméthylène ou du poly(oxyde d'hexaméthylène).

12. Dérivation cardiaque suivant l'une quelconque des revendications 6 à 11, dans laquelle les segments durs comprennent un diisocyanate et un diol.

13. Procédé de fixation d'une tubulure thermoplastique de dérivation cardiaque à une électrode ou à un connecteur à broche de dérivation cardiaque muni de moyens de fixation extérieurs thermoplastiques, le procédé comprenant les stades dans lesquels :
a ) on met en position un tronçon d'extrémité de la tubulure coaxialement autour d'au moins une partie des moyens de fixation ;
b ) on chauffe au moins une partie du tronçon d'extrémité de la tubulure et au moins une partie des moyens de fixation de manière à obtenir une fusion de la partie chauffée du tronçon d'extrémité de la tubulure à la partie chauffée des moyens de fixation ; et
c ) on laisse se solidifier le tronçon fondu obtenu.

14. Procédé suivant la revendication 13, dans lequel on effectue le chauffage par conduction, par exposition à un rayonnement ou par des ultrasons.

15. Procédé suivant la revendication 13, dans lequel on effectue le chauffage et la fusion par soudage par la chaleur.

16. Procédé suivant l'une quelconque des revendications 13 à 15, dans lequel les moyens de fixation thermoplastiques sont prévus dans une cavité de l'électrode ou du connecteur à broche.

17. Procédé suivant l'une quelconque des revendications 13 à 16, dans lequel les moyens de fixation thermoplastiques s'étendent sensiblement autour du périmètre de l'électrode ou du connecteur à broche.

18. Procédé suivant l'une quelconque des revendications 13 à 17, dans lequel la matière des moyens de fixation thermoplastiques et/ou de la tubulure thermoplastique comprend un polyuréthane, une polyoléfine, un polycarbonate, un polyester, un polyamide ou un polyimide.

19. Procédé suivant la revendication 18, dans lequel la matière des moyens de fixation thermoplastiques et/ou de la tubulure thermoplastique comprend un polyéther polyuréthane, un polycarbonate polyuréthane, un poly(siloxane-carbonate) polyuréthane ou un poly(éther-siloxane) polyuréthane.

20. Procédé suivant l'une quelconque des revendications 13 à 19, dans lequel la matière de la tubulure thermoplastique comprend un polyéther polyuréthane ayant des segments souples et des segments durs.

21. Procédé suivant la revendication 20, dans lequel les segments souples comprennent du poly(oxyde de tétraméthylène) ou du poly(oxyde d'hexaméthylène).

22. Procédé suivant l'une quelconque des revendications 13 à 19, dans lequel la matière de la tubulure thermoplastique comprend du poly(éther siloxane) polyuréthane comprenant des segments souples et des segments durs.

23. Procédé suivant la revendication 22, dans lequel les segment souples comprennent du poly(oxyde d'hexaméthylène ) et du polydiméthylsiloxane.

24. Procédé suivant l'une quelconque des revendications 13 à 23, dans lequel la matière des moyens de fixation thermoplastiques comprend un polyéther polyuréthane comprenant des segments souples et des segments durs.

25. Procédé suivant la revendication 24, dans lequel les segments souples comprennent du poly(oxyde de tétraméthylène) ou du poly(oxyde d'hexaméthylène).

26. Procédé suivant l'une quelconque des revendications 20 à 25, dans lequel les segments durs comprennent un diisocyanate et un diol.
